Europäisches Patentamt

European Patent Office

Office européen des brevets

(19)

(11) Publication number: **0 022 292**
**B1**

(12) **EUROPEAN PATENT SPECIFICATION**

(45) Date of publication of patent specification: **11.05.83**

(51) Int. Cl.³: **C 07 D 201/08,**
**C 07 D 207/26**

(21) Application number: **80200617.1**

(22) Date of filing: **28.06.80**

(54) **Process for the preparation of a 2-pyrrolidone.**

(30) Priority: **07.07.79 NL 7905326**

(43) Date of publication of application:
**14.01.81 Bulletin 81/2**

(45) Publication of the grant of the patent:
**11.05.83 Bulletin 83/19**

(84) Designated Contracting States:
**BE DE FR GB IT NL**

(56) References cited:
**GB - A - 1 522 725**
**US - A - 4 123 438**

(73) Proprietor: **STAMICARBON B.V.**
**Postbus 10**
**NL-6160 MC Geleen (NL)**

(72) Inventor: **de Man, Hendrikus C.J.**
**Irenelaan 22**
**NL-6165 CP Geleen (NL)**
Inventor: **Corvers, Antonius**
**Kelmonderstraat 33**
**NL-6191 RD Beek (L.) (NL)**
Inventor: **Thomissen, Petrus J. H.**
**Planetenhof 41**
**NL-6215 TT Maastricht (NL)**

(74) Representative: **Pinckaers, August René et al,**
**OCTROOIBUREAU DSM Postbus 9**
**NL-6160 MA Geleen (NL)**

Process for the preparation of a 2-pyrrolidone

The invention relates to a process for the preparation of a 2-pyrrolidone by catalytic hydrogenation of succinonitrile, optionally substituted, in the liquid phase in the presence of ammonia and treatment of the hydrogenated product obtained with water.

This known process is described in U.S. Patent Specification 4.123.438. This Patent Specification reports a yield of 86% of the theoretical possible yield in carrying out the hydrogenation with a catalyst suspended in the liquid phase at a partial hydrogen pressure of between 1 and 50 atmospheres.

An embodiment of this hydrogenation has now been found whereby a yield of above 86% can be obtained, at partial hydrogen pressures above 50 atmospheres (5000 kPa).

The process according to the invention for the preparation of a 2-pyrrolidone by catalytic hydrogenation of succinonitrile, optionally substituted, in the liquid phase with the aid of a catalyst suspended in the liquid phase in the presence of ammonia and treatment of the hydrogenated product obtained with water is characterized in that the succinonitrile, dissolved in liquid ammonia, is subjected to the hydrogenation and the hydrogenation is carried out at a partial hydrogen pressure of 5500—10000 kPa in a continuous manner in two or more stirred reactors connected in series.

By the process according to the invention, succinonitrile or a substituted succinonitrile can be used as feedstock, such as for instance succinonitrile substituted in the 2nd and/or 3rd position with an alkyl group of 1—4 carbon atoms.

The concentration of the succinonitrile in the liquid ammonia may be variously chosen, for example between 1 and 250 gram of the succinonitrile per 100 gram liquid ammonia. Preferably, a quantity of 5 to 100 gram of the succinonitrile per 100 gram liquid ammonia is applied. If so desired, in addition to liquid ammonia, a minor amount of another solvent (at most 1 gram per gram of succinonitrile) can also be applied such as for instance, benzene, toluene, xylene, dioxane, tetrahydrofurane, pyridine and pyrrolidone. The presence of such another solvent does not have any advantage, however.

The hydrogenation according to the invention can be carried-out at various temperatures, for example a temperature between 50 and 150°C. Temperatures between 60 and 120°C are very suitable.

The partial hydrogen pressure in the hydrogenation according to the invention can also be varied between 1 and 300 bar, for example. In practice, a very favourable result can be achieved at a partial hydrogen pressure of between 500 and 10000 kPa, in particular 5500—10000 kPa.

As in the known process, in the process according to the invention any hydrogenation catalyst may in principle be applied such as for example Raney nickel, Raney cobalt, palladium on carbon and nickel on silica. Preferably, a nickel-containing catalyst, say Raney nickel, is applied, because the activity and selectivity can then be maintained at a high level for a long time.

The hydrogenation according to the invention is performed in 2 or more stirred reactors. In practice, a suitable result can be obtained with 3 stirred reactors if the stirring energy usual in liquid-phase hydrogenation is fed to the reaction liquid. More than 3 stirred reactors may also be used, but this gives no additional advantage.

After the hydrogenation the ammonia may be wholly or partially eliminated. The treatment of the hydrogenated product with water may if so desired, be carried out in the presence of ammonia. Various temperatures may be applied in this water treatment, for example temperatures of between 150 and 300°C. The quantity of water may be variously chosen, for example a quantity of water of 1—25 mol water per mol of the succinonitrile.

The process according to the invention is further elucidated in the following example.

Example

In 3 series-connected reactors, each with a capacity of 2 litres (diameter 125 mm) and provided with a turbine stirrer (diameter 55 mm), a heating jacket and a reflux cooler, succinonitrile, dissolved in liquid ammonia, is hydrogenated in a continuous manner at a hydrogen pressure of 7700 kPa and a temperature of about 75°C. In every reactor 8 gram Raney nickel is used as catalyst and a filter candle is present through which the reaction liquor is discharged. Periodically, a short pressure shock is applied to feed the quantity of catalyst collected on the filter candle back to the reactor. The reactors are stirred at a rate of 2000 rpm. To the first reactor 223 gram succinonitrile, dissolved in 902 gram liquid ammonia, are fed per hour. The volume of the reaction mixture in each reactor is about 1,5 litres. After passing through the third reactor, the reaction mixture obtained is expanded and the ammonia evaporated. During a stationary period of 8 hours, a total of 1873 gram hydrogenated product is obtained. Of the hydrogenated product 150 gram together with 150 gram water is introduced into a steel tube and heated at 210°C for 2 hours. The product obtained is gas-chromatographically analyzed and found to contain 142 gram 2-pyrrolidone. The yield is thus 94% of the theoretical possible yield.

## Claims

1. Process for the preparation of a 2-pyrrolidone by catalytic hydrogenation of optionally substituted succinonitrile, in the liquid phase with the aid of a catalyst suspended in the liquid phase in the presence of ammonia and treatment of the obtained hydrogenated product with water, characterized in that the succinonitrile, dissolved in liquid ammonia, is subjected to the hydrogenation and the hydrogenation is carried out at a partial hydrogen pressure of 5500—10000 kPa in a continuous manner in two or more series-connected stirred reactors.

2. Process according to Claim 1, characterized in that 5—100 gram of the succinonitrile is used per 100 gram liquid ammonia.

3. Process according to Claims 1 or 2, characterized in that the hydrogenation is carried out at a temperature of between 60 and 120°C.

4. Process according to any one of Claims 1—3, characterized in that the hydrogenation is carried out with a nickel-containing catalyst.

## Patentansprüche

1. Verfahren zur Herstellung eines 2-Pyrrolidons durch katalytische Hydrierung eines gegebenenfalls substituierten Bernsteinsäurenitrils in der Flüssigphase mit Hilfe eines in der Flüssigphase suspendierten Katalysators in Gegenwart von Ammoniak und Behandlung des erhaltenen hydrierten Produkts mit Wasser, dadurch gekennzeichnet, dass das in flüssigem Ammoniak gelöste Bernsteinsäurenitril hydriert wird und dass die Hydrierung bei einem Wasserstoff-Partialdruck von 5500—10000 kPa im Dauerverfahren in zwei oder mehr in Reihe geschalteten Rühr-Reaktoren erfolgt.

2. Verfahren nach Anspruch 1, dadurch gekennzeichnet, dass 5—100 Gramm des Bernsteinsäurenitrils je 100 Gramm flüssiges Ammoniak verwendet werden.

3. Verfahren nach Anspruch 1 oder 2, dadurch gekennzeichnet, dass die Hydrierung bei einer Temperatur zwischen 60 und 120°C durchgeführt wird.

4. Verfahren nach einem der Ansprüche 1—3, dadurch gekennzeichnet, dass die Hydrierung mit einem Nickel enthaltenden Katalysator durchgeführt wird.

## Revendications

1. Procédé de préparation de pyrrolidone-2 par l'hydrogénation catalytique de succinonitrile, éventuellement substitué, en phase liquide, à l'aide d'un catalyseur mis en suspension dans la phase liquide et en présence d'ammoniac, alors que le produit hydrogéné obtenu est traité avec de l'eau caractérisé en ce que le succinonitrile, dissous dans de l'ammoniac liquide, est soumis à l'hydrogénation et que celle-ci est effectuée de façon continue à une pression partielle d'hydrogène de 5500 à 10000 kPa, dans deux ou plusieurs réacteurs sous agitation accouplés en série.

2. Procédé selon la revendication 1, caractérisé en ce qu'on utilise 5—100 g de succinonitrile par 100 g d'ammoniac liquide.

3. Procédé selon l'une des revendications 1 et 2, caractérisé en ce que l'hydrogénation est effectuée à une température située entre 60 et 120°C.

4. Procédé selon l'une des revendications 1 à 3, caractérisé en ce que l'hydrogénation est effectuée à l'aide d'un catalyseur contenant du nickel.